Europäisches Patentamt

European Patent Office

Office européen des brevets

(19)

(11) **EP 0 677 047 B1**

(12) **EUROPÄISCHE PATENTSCHRIFT**

(45) Veröffentlichungstag und Bekanntmachung des Hinweises auf die Patenterteilung:
**05.02.1997   Patentblatt 1997/06**

(51) Int Cl.6: **C07D 303/04**, C07D 301/10, B01J 38/16

(21) Anmeldenummer: **94901917.8**

(22) Anmeldetag: **30.11.1993**

(86) Internationale Anmeldenummer:
**PCT/EP93/03352**

(87) Internationale Veröffentlichungsnummer:
**WO 94/13653 (23.06.1994 Gazette 1994/14)**

(54) **VERFAHREN ZUR HERSTELLUNG VON 3,4-EPOXY-1-BUTEN**

METHOD OF PREPARING 3,4-EPOXY-1-BUTENE

PROCEDE DE PREPARATION DE 3,4-EPOXY-1-BUTENE

(84) Benannte Vertragsstaaten:
**AT BE CH DE ES FR GB IT LI LU NL SE**

(30) Priorität: **11.12.1992   DE 4241942**

(43) Veröffentlichungstag der Anmeldung:
**18.10.1995   Patentblatt 1995/42**

(73) Patentinhaber: **BASF Aktiengesellschaft
67063 Ludwigshafen (DE)**

(72) Erfinder:
  • **BOECK, Stefan
    D-6700 Ludwigshafen (DE)**
  • **HERZOG, Klaus
    D-6700 Ludwigshafen (DE)**
  • **FISCHER, Rolf
    D-6900 Heidelberg (DE)**
  • **VOGEL, Herbert
    D-6700 Ludwigshafen (DE)**
  • **FISCHER, Martin
    D-6700 Ludwigshafen (DE)**

(56) Entgegenhaltungen:
**EP-A- 0 326 392          FR-A- 2 342 791
US-A- 5 117 012**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von 3,4-Epoxy-1-buten durch die Gasphasenepoxidierung von 1,3-Butadien mittels Sauerstoff oder sauerstoffhaltigen Gasen an silberhaltigen Katalysatoren und Isolierung des 3,4-Epoxy-1-butens aus dem Reaktionsaustrag.

3,4-Epoxy-1-buten, auch als Butadienmonoxid oder als Vinyloxiran bezeichnet, ist ein Zwischenprodukt zur Herstellung von beispielsweise Tetrahydrofuran, das daraus z.B. nach dem Verfahren von US-A 5 034 545 durch die Isomerisierung zu 2,5-Dihydrofuran und dessen nachfolgende Hydrierung hergestellt werden kann. Außerdem dient es zur Herstellung von 1,2-Butylenoxid (vgl. US-A 5 117 013). Für eine wirtschaftliche Produktion der zuvor genannten, in großen Mengen benötigten Chemikalien auf der Basis des Ausgangsmaterials 3,4-Epoxy-1-buten ist ein leistungsfähiges, wirtschaftliches Verfahren zur Herstellung von 3,4-Epoxy-1-buten erforderlich. Ein derartiges Verfahren ist aber bislang nicht verfügbar.

So beschreibt WO 89/07101 ein Verfahren zur Gasphasenepoxidierung von 1,3-Butadien mit Sauerstoff oder sauerstoffhaltigen Gasen an silberhaltigen Katalysatoren. Die dabei erzielten Ausbeuten und Selektivitäten sind anfangs recht gut, allerdings lassen die dabei verwendeten silberhaltigen Katalysatoren sehr schnell in ihrer Aktivität nach und verlieren innerhalb 24 Stunden nahezu 90 % ihrer ursprünglichen Aktivität. Somit ist dieses Verfahren für die Herstellung von 3,4-Epoxy-1-buten im industriellen Maßstab ungeeignet.

Vermutlich wird diese Desaktivierung durch die Belegung der Katalysatoroberfläche mit Zersetzungsprodukten des Vinyloxirans verursacht, ein Vorgang, der im folgenden als Verkokung bezeichnet wird. Unter Desaktivierung wird im folgenden, falls nicht anders angemerkt, die Desaktivierung des Silberkatalysators durch Verkokungsprozesse bei der Vinyloxiranerzeugung verstanden, die zu einer raschen Aktivitätsabnahme, d.h. zu einer Aktivitätsabnahme um mehr als 50 % der Anfangsaktivität innerhalb weniger Stunden oder Tage, des silberhaltigen Katalysators führt. Diese Art der Desaktivierung ist von der normalen Desaktivierung des silberhaltigen Katalysators infolge von erst im Laufe von Monaten und Jahren in Erscheinung tretenden Alterungsprozessen, wie thermischen Schädigungen des Katalysators, Sinterungsvorgängen, Phasenentmischungen oder dem allmählichen Austrag von Katalysatorbestandteilen, zu unterscheiden.

US-A 5 117 012 betrifft ein Verfahren zur Aufarbeitung des gasförmigen Reaktionsaustrags aus der Gasphasenepoxidierung von 1,3-Butadien nach dem Verfahren von WO 89/07101, in dem der heiße, gasförmige Reaktionsaustrag mit Hilfe von flüssigem 1,3-Butadien abgeschreckt wird. In dieser Schrift wird erwähnt, daß der Reaktorzulauf aus 1,3-Butadien, Sauerstoff und Inertgas weniger als 5 mol-%, bezogen auf den gesamten Zulauf, an Wasser enthalten soll, da andernfalls mit erheblichen Verlusten an 3,4-Epoxy-1-buten infolge Diolbildung zu rechnen sei.

FR-A 2 342 791 betrifft ein Verfahren für die Wiederherstellung der Selektivität von in Verfahren zur Herstellung von Ethylenoxid gebrauchten Alkalimetall-dotierten Silberkatalysatoren mit durch diesen Gebrauch verringerter Selektivität, in dem diese Silberkatalysatoren mit Wasserdampf befeuchtet und anschließend bei Temperaturen von 100 bis 300°C getrocknet werden.

Der vorliegenden Erfindung lag nunmehr die Aufgabe zugrunde, ein Verfahren zur Gasphasenepoxidierung von 1,3-Butadien mittels Sauerstoff oder sauerstoffhaltigen Gasen zu finden, das es ermöglicht, 3,4-Epoxy-1-buten im industriellen Maßstab auf wirtschaftliche Weise herzustellen, wobei insbesondere die Aktivität der dazu eingesetzten Katalysatoren langfristig erhalten bleiben sollte.

Dementsprechend wurde ein Verfahren zur Herstellung von 3,4-Epoxy-1-buten durch die Gasphasenepoxidierung von 1,3-Butadien mittels Sauerstoff oder sauerstoffhaltigen Gasen an silberhaltigen Katalysatoren und Isolierung des 3,4-Epoxy-1-butens aus dem Reaktionsaustrag gefunden, das dadurch gekennzeichnet ist, daß man die Gasphasenepoxidierung in Gegenwart von 6 bis 80 mol-% Wasserdampf, bezogen auf das dem Reaktor zugeführte Gasgemisch, durchführt.

Des weiteren wurde ein Verfahren zur Reaktivierung von zur Herstellung von 3,4-Epoxy-1-buten durch die Gasphasenepoxidierung von 1,3-Butadien mittels Sauerstoff oder sauerstoffhaltigen Gasen verwendeter, durch Verkokung desaktivierter, silberhaltiger Katalysatoren gefunden, das dadurch gekennzeichnet ist, daß man die desaktivierten, silberhaltigen Katalysatoren bei Temperaturen von 100 bis 400°C mit Wasserdampf und Sauerstoff oder Sauerstoff enthaltenden Gasen behandelt.

Das erfindungsgemäße Verfahren ermöglicht überraschenderweise den langfristigen Betrieb der Gasphasenepoxidierung von 1,3-Butadien mit Hilfe silberhaltiger Katalysatoren, ohne daß es zu nennenswerten Aktivitätsverlusten der silberhaltigen Katalysatoren infolge Verkokung kommt. Darüber hinaus ermöglicht es das erfindungsgemäße Verfahren, bei bereits desaktivierten Katalysatoren einen Großteil ihrer ursprünglichen Aktivität bei der Gasphasenepoxidierung von 1,3-Butadien zu reaktivieren.

Die Ursache für diese überraschenden Effekte eines Wasserzusatzes zum gasförmigen Zulauf des Epoxidierungsreaktors ist noch nicht geklärt. Erstaunlicherweise bleibt selbst im Falle eines hohen Wasserzusatzes zum gasförmigen Zulauf des Epoxidierungsreaktors, die hohe Selektivität des silberhaltigen Katalysators bezüglich der Erzeugung von Vinyloxiran erhalten.

Der Wasserzusatz zum gasförmigen Zulauf des Epoxidierungsreaktors beträgt im allgemeinen 6 bis 80 mol-%, vorteilhaft 8 bis 70 mol-% und besonders bevorzugt 10 bis 50 mol-%, bezogen auf den gesamten, gasförmigen Zulauf zum Reaktor. Bei der Angabe des Wasserzusatzes in mol-% wird davon ausgegangen, daß sich die Gase im Gasgemisch annähernd wie ideale Gase verhalten, so daß die Angabe in mol-% praktisch einer Angabe in Vol.-% entspricht. Zweckmäßigerweise wird das Wasser in Form von Dampf zum gasförmigen Zulauf dosiert, der Zusatz des Wassers mittels Zerstäubern oder anderen Vorrichtungen zur Einbringung von Wasser in einen Gasstrom ist ebenfalls möglich.

Zur Durchführung des erfindungsgemäßen Verfahrens wird ein Gasstrom, bestehend aus 1,3-Butadien, Sauerstoff, dem Wasser und gewünschtenfalls unter den angewandten Reaktionsbedingungen inerten, gasförmigen Verdünnungsmitteln, wie Stickstoff, Argon, Kohlenwasserstoffen, wie Methan oder Ethan und/oder Kohlendioxid und/oder Reaktionsmoderatoren, wie Stickstoffoxiden und/oder Halogenalkanen mit mindestens einem Wasserstoffatom im Molekül, wie Methylchlorid, Methylbromid, Dichlormethan, Dibrommethan, Chloroform, Bromoform, Ethylchlorid, Ethylbromid, Dichlorethan, Dibromethan, Vinylchlorid, Dichlorethylen, Trichlorethylen, Dichlor) propan, Dibrompropan, Dichlorpropen, Dibrompropen, Chlorbutan usw., in den Reaktor geleitet. In der Regel wird dabei ein Butadien/Sauerstoff-Molverhältnis von 0,05 bis 40, vorzugsweise von 0,1 bis 25 und insbesondere von 0,3 bis 15 eingestellt. Der Volumenanteil von Butadien im Zulauf zum Reaktor beträgt im allgemeinen 5 bis 80 Vol.-%, vorzugsweise 10 bis 70 Vol.-% und besonders bevorzugt 15 bis 45 Vol.-% und der Volumenanteil des Sauerstoffs im Zulauf zum Reaktor beträgt im allgemeinen 2 bis 80 Vol.-%, vorzugsweise 3 bis 70 Vol.-% und besonders bevorzugt 5 bis 45 Vol.-%.

Bevorzugte Inertgase sind Stickstoff sowie die $C_1$- bis $C_4$-Alkane, besonders bevorzugt werden die $C_1$- bis $C_4$-Alkane, insbesondere das Methan, als inerte, gasförmige Verdünnungsmittel benutzt. Zweckmäßigerweise wird das betreffende Inertgas oder ein Gemisch aus zweien oder mehreren unter den Reaktionsbedingungen inerten Gasen, vorzugsweise aus zweien oder mehreren der zuvor genannten Inertgase, besonders bevorzugt die genannten Alkane und insbesondere das Methan, dem Gaszulauf in solchen Mengen zugesetzt, daß das dem Reaktor zugeführte Gasgemisch 2 bis 87 Vol.-%, vorzugsweise 5 bis 79 Vol.-% und besonders bevorzugt 10 bis 70 Vol.-% des betreffenden Inertgases enthält. Es wurde festgestellt, daß durch die Zugabe der genannten Kohlenwasserstoffe zum Gaszulauf des erfindungsgemäßen Verfahrens, das Sauerstoff/Butadien-Molverhältnis auf höhere Werte als bei alleiniger Verwendung des Inertgases Stickstoff eingestellt werden kann, ohne daß dieses Gemisch zündfähig, d.h. explosiv, wird. Dieser Effekt führt neben wirtschaftlichen Vorteilen zu einem beträchtlichen Gewinn an Sicherheit bei der Betreibung des erfindungsgemäßen Verfahrens.

Die Reaktionsmoderatoren, deren Zusatz zur Inhibierung der Weiteroxidation des Vinyloxirans zu Kohlendioxid und Wasser dient, werden dem Gasstrom im allgemeinen in Mengen von 0 bis 10.000 mol-ppm, vorzugsweise von 0,1 bis 1000 und insbesondere von 0,2 bis 100 mol-ppm, bezogen auf die gesamte Gasmischung, zudosiert.

Dieser Gaszulauf wird im allgemeinen bei Temperaturen von 100°C bis 400°C, vorzugsweise von 120 bis 350°C und besonders bevorzugt von 150 bis 300°C und bei einem Druck von 0,1 bis 100 bar, vorteilhaft von 0,5 bis 50 bar, insbesondere von 1 bis 30 bar über den silberhaltigen Katalysator geleitet und partiell oxidiert. Unter den angewandten Bedingungen sind die Reaktionsgase Butadien, Wasser und Vinyloxiran im gasförmigen oder überkritischen, fluiden Zustand. Die Raumgeschwindigkeit des Gaszulaufs beträgt bei der bevorzugten, kontinuierlichen Betriebsweise des Verfahrens im allgemeinen 20 bis 20.000 $h^{-1}$, vorzugsweise von 50 bis 15.000 $h^{-1}$ und besonders bevorzugt von 100 bis 10.000 $h^{-1}$.

Der Umsatz des Butadiens wird in der Regel auf 0,5 bis 100 mol-%, vorteilhaft auf 2 bis 80 mol-% und besonders bevorzugt auf 5 bis 20 mol-% der gesamten zugeführten 1,3-Butadienmenge eingestellt. Der Umsatz des Butadiens kann über die Raumgeschwindigkeit des Gaszulaufs, die Temperatur der Katalysatorschüttung und durch den Zusatz der halogenhaltigen Reaktionsmoderatoren gesteuert werden.

Vorzugsweise wird das erfindungsgemäße Verfahren kontinuierlich betrieben, wobei vorteilhaft Rohr- oder Rohrbündelreaktoren, in denen der Katalysator vorteilhaft in einem Festbett angeordnet ist, verwendet werden können. Diese Reaktoren werden vorzugsweise isotherm betrieben, wobei für derartige Zwecke übliche Wärmeträger, z.B. Wasser, Kohlenwasserstoffe, wie Kerosin, Naphthalin oder Biphenyl, und Salzschmelzen verwendet werden können. Wasser wird in der Regel als Wärmeträger bevorzugt eingesetzt.

Bei der isothermen Betriebsweise kann der silberhaltige Katalysator im Festbett sowohl in einer losen Katalysatorschüttung, die von den Wärmeträgerrohren durchzogen wird, als auch in Rohren, die von außen thermostatisiert werden, angeordnet werden.

Zur Aufarbeitung wird das heiße, die Katalysatorschüttung verlassende Reaktionsgas, das Vinyloxiran, nicht umgesetztes 1,3-Butadien, gegebenenfalls Inertgase und/oder Reaktionsmoderatoren enthält, abgekühlt, beispielsweise mittels direktem Wärmeaustausch, z.B. durch Einspritzen eines kühlen Lösungsmittels oder durch Eindüsen eines kühlen Gases, wie Stickstoff, Luft, Kohlendioxid usw., in das heiße Reaktionsgas oder vorzugsweise mittels indirektem Wärmeaustausch, z.B. durch herkömmliche Kühler oder Wärmeaustauscher, wobei zweckmäßigerweise das heiße Reaktionsgas zur Vorwärmung des Reaktorzulaufs genutzt wird. Nach der Abkühlung des Reaktionsgases kann das Vinyloxiran in geeigneter Weise, z.B. durch Kondensation, oder bevorzugt durch eine Wäsche des Reaktionsgases in einem Gaswäscher mit einem geeigneten Lösungsmittel aus dem Gasstrom entfernt werden. Als Lösungsmittel wählt man zu diesem Zweck vorteilhaft solche Lösungsmittel aus, aus denen das Vinyloxiran und/oder das 1,3-Butadien auf einfache Art und Weise abgetrennt werden können. Ein geeignetes derartiges Lösungsmittel ist z.B. 1,3-Butadien selbst (vgl. US-A 5 117 012), besonders bevorzugt wird Wasser zum Auswaschen des Vinyloxirans aus dem Reaktionsgas verwendet. Während sich Vinyloxiran in Wasser löst, ist 1,3-Butadien in Wasser praktisch unlöslich. In einem Gas-Flüssig-Abscheider können das wasserhaltige, gasförmige 1,3-Butadien und die anderen wasserunlöslichen Bestandteile des Reaktionsgases von der vinyloxiranhaltigen, wäßrigen Phase abgetrennt und wieder in den Reaktor zurückgeführt werden. Aus der wäßrigen Phase kann das Vinyloxiran auf einfache Weise, z.B. durch Durchleiten von Wasserdampf, Luft, Stickstoff, Kohlendioxid und/oder anderen gegenüber Vinyloxiran unter diesen Bedingungen inerten Gasen ausgetrieben und gegebenenfalls nach einer weiteren destillativen Reinigung in reiner Form isoliert werden.

Die Temperatur des Wassers zur Extraktion des Vinyloxirans aus dem Reaktionsaustrag wird im allgemeinen auf Temperaturen bis 100°C, vorzugsweise auf Temperaturen bis 80°C, insbesondere auf Temperaturen bis 60°C eingestellt. Zur Extraktion des Vinyloxirans aus dem Reaktionsaustrag können an sich herkömmliche Vorrichtungen verwendet werden, wie sie zur Extraktion oder Absorption von Gasen benutzt werden, z.B. Extraktionskolonnen.

Das als Nebenprodukt bei der Herstellung von Vinyloxiran entstehende Kohlendioxid kann gewünschtenfalls durch eine Wäsche aus dem von Vinyloxiran befreiten Reaktionsaustrag mit Hilfe geeigneter Lösungs- oder Absorptionsmittel, insbesondere basisch wirkender Absorptionsmittel, wie N-Methylpyrrolidon, N-Methyldiethanolamin, N-Methylethanolamin, oder wäßriger Alkalimetallhydrogencarbonatlösungen, extrahiert werden. Gewünschtenfalls kann das Kohlendioxid aus diesen Absorptionsflüssigkeiten anschließend wieder desorbiert werden, z.B. durch Erwärmung oder Abtreiben mit Dampf oder Inertgasen, und weiterverwendet werden.

Das solchermaßen vom Vinyloxiran und Kohlendioxid befreite Gasgemisch, das im wesentlichen aus 1,3-Butadien, Wasserdampf und Inertgasen besteht, wird vorteilhaft wieder in den Reaktor zur Herstellung von Vinyloxiran zurückgeführt, wobei der Gehalt des rückgeführten Gases, im folgenden als Kreisgas bezeichnet, an 1,3-Butadien, Wasserdampf, Sauerstoff, Inertgas und Reaktionsmoderatoren zweckmäßigerweise durch Zudosieren dieser Gase auf einen für die Herstellung von Vinyloxiran optimalen Gehalt eingestellt wird. Zur Vermeidung der Anreicherung von Nebenprodukten im Kreisgas kann es vorteilhaft sein, einen kleinen Anteil des Kreisgases, im allgemeinen nicht mehr als 15 Vol.-% aus dem Kreisgasstrom auszuschließen.

Als Katalysatoren werden im erfindungsgemäßen Verfahren Silberkatalysatoren verwendet, die 0,1 bis 50 Gew.-% Silber, vorteilhaft 1 bis 30 Gew.-% und besonders bevorzugt 2 bis 20 Gew.-% Silber, berechnet als Ag und bezogen auf das Gesamtgewicht des Katalysators, auf einem Trägermaterial enthalten. Pures Silber, z.B. Silberkristallpulver oder Elektrolytsilber, kann ebenfalls verwendet werden.

Als Trägermaterialien für derartige silberhaltige Katalysatoren können eine Vielzahl von Trägermaterialien wie Siliciumdioxid, Aluminiumoxide, Silicium-Aluminium-Mischoxide, Titanoxide, Lanthanoxid, Magnesiumoxid, Bornitrid, Borcarbid, Siliciumnitrid, Siliciumcarbid, Zinkoxid, Zinnoxide, Eisenoxide, Calciumoxid, Bariumoxid, Strontiumoxid, Zirkoniumdioxid, Kohlenstoff, Borphosphat, Zirkoniumphosphat, Thoriumoxid, Galliumoxid, Indiumoxid oder ähnliche Trägermaterialien, allein oder in Gemischen mit anderen Trägermaterialien dienen.

Im allgemeinen werden Trägermaterialien benutzt, die eine BET-Oberfläche von weniger als 50 $m^2/g$, vorzugsweise weniger als 10 $m^2/g$ und insbesondere weniger als 2 $m^2/g$ haben. Besonders bevorzugte Trägermaterialien umfassen die Aluminiumoxide, insbesondere das $\alpha$-Aluminiumoxid, Zirkoniumdioxid, Mischungen aus $\alpha$-Aluminiumoxid und Zirkoniumdioxid, Titandioxid, Siliciumdioxid und Siliciumcarbid. Die Porosität dieser Trägermaterialien beträgt im allgemeinen 5 bis 90 %, vorzugsweise 10 bis 80 %, gemessen nach dem Verfahren der Quecksilberporosimetrie.

Die äußere Form der Katalysatorträger ist in der Regel im erfindungsgemäßen Verfahren für die Katalysatoraktivität nicht kritisch. So können z.B. Träger in Form von Kugeln, Zylindern, Ringen, Sattelkörpern, Spiralen oder anderen Formgebungen eingesetzt werden, vorzugsweise werden Ringe, Kugeln oder Sattelkörper benutzt, die in der Regel eine ausreichende geometrische Oberfläche haben und dabei einen nur niedrigen Druckverlust des die Katalysatorschüttung durchströmenden Reaktionsgases verursachen.

Vorzugsweise werden im erfindungsgemäßen Verfahren solche Silberkatalysatoren verwendet, die außer dem Silber und dem Trägermaterial noch 0,001 bis 10 Gew.-% an Promotoren enthalten. Geeignete Promotoren sind z.B. Alkalimetalle und Erdalkalimetalle, die Seltenerdmetalle, die Metalle der IV., V., VI., VII., VIII. und II. Nebengruppe des Periodensystems der Elemente sowie Kupfer, Gold und Thallium. Besonders bevorzugte Promotoren sind die Alkalimetalle und Erdalkalimetalle, insbesondere die schweren Alkalimetalle Kalium, Rubidium und Cäsium sowie die Elemente der VI. und VII. Nebengruppe des Periodensystems der Elemente, insbesondere Molybdän, Wolfram und Rhenium. Die chemische Form, in der diese Promotoren im oder auf dem silberhaltigen Katalysator vorliegen oder wirken, ist noch nicht bekannt.

Im allgemeinen werden diese Promotoren in Form ihrer Salze, insbesondere ihrer Halogenide, Nitrate, Carboxylate, Sulfate, Carbonate oder Phosphate, ihrer Oxide oder Hydroxide auf das betreffende Trägermaterial aufgebracht. Die Art der Anionen der Salze der verwendeten Promotoren ist im allgemeinen von geringerer Bedeutung für die katalytische Aktivität der mit den Promotoren dotierten Silberkatalysatoren. Vorzugsweise werden solche Salze, Oxide oder Hydroxide zur Dotierung der im erfindungsgemäßen Verfahren verwendbaren Silberkatalysatoren eingesetzt, die in den zur Tränkung des Katalysators verwendeten Lösungsmitteln löslich sind. Es können auch Komplexe oder Komplexsalze der betreffenden Promotoren verwendet werden, beispielsweise Alkalimetall-Molybdate, Alkalimetall-Wolframate oder Alkalimetall-Rhenate oder Perrhenate.

Eine beispielhafte Aufzählung von Verbindungen, die als Promotoren zur Dotierung der erfindungsgemäß einsetzbaren Katalysatoren benutzt werden können, wird im folgenden gegeben: Lithiumchlorid, Lithiumbromid, Lithiumsulfat, Lithiumnitrat, Lithiumcarbonat, Lithiumphosphat, Lithiumhydroxid, Lithiumoxid, Lithiummolybdat, Lithiumwolframat, Lithiumrhenat, Lithiumperrhenat, Lithiumacetat, Lithiumformiat, Lithiumcitrat, Lithiumoxalat usw. sowie die entsprechenden Salze, Hydroxide und Oxide des Natriums, Kaliums, Rubidiums und Cäsiums, Magnesiumnitrat, Magnesiumsulfat, Magnesiumformiat, Magnesiumchlorid, Magnesiumacetat, Magnesiumcitrat usw., Calciumchlorid, Calciumbromid, Calciumhydroxid, Calciumcitrat, Calciumacetat, Calciumnitrat usw., Bariumchlorid, Bariumoxid, Bariumhydroxid, Bariumnitrat, usw. Besonders bevorzugt werden Alkalimetallsalze, Alkalimetallhydroxide und/oder -oxide gegebenenfalls zusammen mit Wolfram-, Molybdän- oder Rheniumverbindungen als Promotoren verwendet.

Das Silber und die Promotoren können bei der Herstellung der im erfindungsgemäßen Verfahren verwendbaren silberhaltigen Katalysatoren nach den herkömmlichen Verfahren zur Herstellung von Katalysatoren auf das Trägermaterial aufgebracht worden sein, beispielsweise durch Auffällen des Silbers und der Promotoren auf den Träger, durch Tränkung des Trägers, durch gemeinsame Fällung des Silbers und der anderen Promotoren mit dem Trägermaterial usw. Die Reihenfolge, in der das Silber und die Promotoren auf dem Trägermaterial abgeschieden werden kann beliebig gewählt werden, insbesondere bei der Herstellung der erfindungsgemäß verwendbaren Katalysatoren durch Imprägnierung des Trägermaterials, kann es unter Umständen vorteilhaft sein, das Silber und die Promotoren gemeinsam in einer Stufe auf das Trägermaterial aufzutränken oder Silber und Promotoren getrennt, in zwei Stufen auf den Träger abzuscheiden. Dabei können der oder die Promotoren vor oder nach dem Silber auf den Träger aufgebracht werden. Andere Variationen der Reihenfolge, in der die einzelnen Katalysatorbestandteile auf dem Träger abgeschieden werden, sind ebenfalls möglich.

Zur Herstellung der im erfindungsgemäßen Verfahren verwendbaren Katalysatoren können praktisch alle Silberverbindungen benutzt werden. Da die silberhaltigen Katalysatoren vorzugsweise nach dem Imprägnierverfahren hergestellt werden, sind im allgemeinen solche Silberverbindungen als Silberquelle bevorzugt, die sich im Imprägniermedium, im allgemeinen Wasser oder polare organische Lösungsmittel, vorzugsweise protische organische Lösungsmittel, losen. Beispiele für solche Silberverbindungen sind Silbernitrat, Silbersulfat, Silberacetat, Silberoxalat und andere Silbercarboxylate. Weiterhin können lösliche Komplexe des Silbers, vorzugsweise Komplexe des Silbers mit stickstoffhaltigen Basen, wie Ammoniak, Hydrazin, Harnstoff, Thioharnstoff, Guanidin oder organischen Aminen, vorzugsweise aliphatischen Aminen, vorteilhaft zur Aufbringung des Silbers verwendet werden. An die Aufbringung des Silbers und der jeweiligen Promotoren in einer oder mehreren Stufen kann sich eine Trocknung des imprägnierten Trägers bei 20 bis 150°C, vorzugsweise bei 50 bis 120°C anschließen, bevor der mit der Silberverbindung getränkte Träger einer thermischen Behandlung bei Temperaturen von 150 bis 600°C, vorzugsweise von 180 bis 400°C zwecks Zersetzung der aufgetränkten Silberverbindungen zu im wesentlichen elementarem Silber unterworfen wird. Zur weiteren Stabilisierung der so hergestellten, silberhaltigen Katalysatoren können diese gewünschtenfalls noch bei 200 bis 800°C, vorzugsweise bei 250 bis 600°C calciniert und/oder im Wasserstoffstrom reduziert werden. Die Art und Weise der Herstellung derartiger Katalysatoren ist an sich bekannt (vgl. WO 89/07101). Geeignete silberhaltige Katalysatoren, die im erfindungsgemäßen Verfahren eingesetzt werden können, sind z.B. die Katalysatoren gemäß WO 89/07101 und US-A 5 081 096.

Der erfindungsgemäße Wasserzusatz zum Gaszulauf ermöglicht es überraschenderweise, das Verfahren zur Herstellung von Vinyloxiran durch die partielle Oxidation von 1,3-Butadien mit molekularem Sauerstoff in der Gasphase (Gasphasenepoxidierung) langfristig zu betreiben, ohne daß es zu einem nennenswerten Aktivitätsverlust des Katalysators infolge Verkokung kommt. Mit Hilfe des erfindungsgemäßen Wasserzusatzes zum Gaszulauf der 1,3-Butadien-Epoxidierung gelingt es sogar, silberhaltigen Katalysatoren, die während ihrer nicht erfindungsgemäßen Verwendung als Katalysator zur Herstellung von Vinyloxiran, innerhalb weniger Stunden ihre katalytische Aktivität infolge Verkokung größtenteils eingebüßt haben, wieder den größten Teil ihrer Katalysatoraktivität zurückzuverleihen, d.h. diese wieder zu reaktivieren.

Es ist aber auch möglich, durch Verkokung desaktivierte silberhaltige Katalysatoren aus der Vinyloxiran-Herstellung in einer separaten Behandlung mit Wasserdampf und Sauerstoff oder Sauerstoff enthaltenden Gasen zu reaktivieren. In der Regel wird diese Reaktivierung chargenweise durchgeführt, eine kontinuierliche Reaktivierung des Katalysators durch dessen Behandlung mit Wasserdampf und Sauerstoff oder Sauerstoff enthaltenden Gasen, z.B. in Wirbelschichtreaktoren oder Drehrohröfen, ist aber ebenfalls möglich. Zur Reaktivierung werden über die bei der Vinyloxiran-Herstellung verkokten Katalysatorchargen im Reaktor, vorzugsweise einem Rohrreaktor, Wasserdampf und Sauerstoff oder Sauerstoff enthaltende Gase bei Temperaturen von im allgemeinen 100 bis 400°C, vorzugsweise von 150 bis 300°C und insbesondere von 180 bis 250°C und einem Druck von im allgemeinen 0,1 bis 100 bar, vorzugsweise von 1 bis 30 bar und insbesondere von 1 bis 20 bar geleitet. Die Zeitdauer dieser Katalysatorreaktivierung hängt in der Regel von der Größe der Katalysatorcharge und vom Grad der Schädigung des Katalysators infolge der Verkokung ab. Der Wassergehalt des zur Reaktivierung der silberhaltigen Katalysatoren eingesetzten Gasgemisches beträgt im allgemeinen 5 bis 95 Vol.-%, vorzugsweise 10 bis 80 Vol.-% und besonders bevorzugt 15 bis 50 Vol.-%, wohingegen der Sauerstoffgehalt des Reaktivierungsgasgemisches in der Regel 5 bis 95 Vol.-%, vornehmlich 10 bis 80 Vol.-% und insbesondere 15 bis 50 Vol.-% beträgt. Zweckmäßigerweise wird das Reaktivierungsgasgemisch mit einer Raumgeschwindigkeit von 20 bis 20 000 h$^{-1}$, vorzugsweise von 50 bis 15 000 h$^{-1}$ und besonders bevorzugt von 100 bis 10 000 h$^{-1}$ über den desaktivierten Katalysator geleitet. Mit Hilfe der erfindungsgemäßen Katalysatorbehandlung kann den desaktivierten, silberhaltigen Katalysatoren aus der Vinyloxiran-Herstellung wieder bis zu 60 % ihrer ursprünglichen Aktivität zurückverliehen werden.

Beispiele

Es wurde in sämtlichen Beispielen ein silberhaltiger Katalysator eingesetzt, der 15,4 Gew.-% Silber, bezogen auf den gesamten Katalysator, auf einem Träger aus $\alpha$-Aluminiumoxid, einer Reinheit von > 98 % enthielt. Zusätzlich war der Katalysator mit 245 Gew.-ppm Lithium und 550 Gew.-ppm Cäsium, bezogen auf den gesamten Katalysator, dotiert worden. Der $\alpha$-Aluminiumoxid-Träger hatte eine BET-Oberfläche von 0,9 m$^2$/g (gemessen nach: Chemie-Ing.-Techn. Bd. 32, 349 (1960); Chemie-Ing.-Techn. Bd. 35, 586 (1963)), eine Wasseraufnahme von 0,47 ml/g (Wasseraufnahme bei 20°C nach 5 Minuten), eine Porosität bzw. einen mittleren Porendurchmesser, beides bestimmt nach dem Verfahren der Quecksilberporosimetrie (Meßgerät: Autopore II, Modell 9220 der Fa. Micromeritics) von 67 % bzw. 10,6 μm und ein Schüttgewicht von 0,63 kg/l. 20 ml des Katalysators wurden in Form von Splitt einer Korngröße von 2,0 bis 2,5 mm in einen stählernen Reaktor gefüllt und bei der gewünschten Raumgeschwindigkeit mit Reaktionsgas durchströmt. Der Reaktor wurde von außen elektrisch beheizt. Das Einsatzgas und das Produktgas wurden gaschromatographisch analysiert. Aus den so erhaltenen Vol.-%-Anteilen der einzelnen Komponenten im Gasgemisch wurde der Umsatz U an 1,3-Butadien und die Selektivität S der Umsetzung zu Butadienmonoxid berechnet:

$$U \ [\%] = \frac{\text{1,3-Butadiengehalt im Zulauf - 1,3-Butadiengehalt im Abgas} \cdot 100}{\text{1,3-Butadiengehalt im Zulauf}}$$

$$S \ [\%] = \frac{\text{Vinyloxirangehalt im Abgas} \cdot 100}{\text{1,3-Butadiengehalt im Zulauf - 1,3-Butadiengehalt im Abgas}}$$

Aufgrund der Fehlerbreite des Analysensystems muß beim Umsatzwert eine Standardabweichung von ± 1 Prozentpunkt und beim Selektivitätswert eine Standardabweichung von ± 2 Prozentpunkten berücksichtigt werden.

Der in den Beispielen verwendete silberhaltige Katalysator wurde nach dem folgenden Verfahren hergestellt: 100 Gew.-teile des Trägermaterials aus $\alpha$-Aluminiumoxid wurden mit einer Lösung getränkt, die folgende Bestandteile enthält: 28,8 Gew.-teile Silbernitrat, 0,290 Gew.-teile Lithiumnitrat, 0,0723 Gew.-teile Cäsiumhydroxid, 25,7 Gew.-teile sek.-Butylamin und 6,3 Gew.-teile Wasser. Anschließend wurde der imprägnierte Träger in einem Bandcalcinierofen unter Stickstoff bei 220°C binnen 10 Minuten zum Katalysator umgewandelt und danach in einem Kammerofen 5 Stunden unter Luft auf 300°C erhitzt.

Beispiel 1 (nicht erfindungsgemäß)

Der Katalysator wurde mit einem Gasgemisch aus 20 Vol.-% 1,3-Butadien und 20 Vol.-% Sauerstoff, Rest Stickstoff, bei einer Temperatur von 225° und bei einem Druck von 1,5 bar mit einer Raumgeschwindigkeit von 1000 h$^{-1}$ belastet. Die Ergebnisse sind in Tabelle 1 aufgelistet.

Beispiel 2 (erfindungsgemäß)

Der nach einer Laufzeit von 24 h desaktivierte Katalysator aus Beispiel 1 wurde mit einer Gasmischung aus 20 Vol.-% 1,3-Butadien, 20 Vol.-% Sauerstoff und 8 Vol.-% Wasserdampf, Rest Stickstoff, bei 225°C und bei einem Druck von 1,5 bar mit einer Raumgeschwindigkeit von 1090 h$^{-1}$ belastet. Die Versuchsergebnisse sind in Tabelle 1 aufgeführt.

Tabelle 1

| Beispiel | Laufzeit [h] | Wasser [%] | U [%] | S [%] |
|---|---|---|---|---|
| 1 | 9 | 0 | 10,8 | 83,0 |
| 1 | 23 | 0 | 1,73 | 83,2 |
| 1 | 24 | 0 | 1,68 | 84,5 |

EP 0 677 047 B1

Tabelle 1   (fortgesetzt)

| Beispiel | Laufzeit [h] | Wasser [%] | U [%] | S [%] |
|---|---|---|---|---|
| 2 | 27 | 8 | 3,97 | 83,9 |
| 2 | 38 | 8 | 6,09 | 80,3 |
| 2 | 66 | 8 | 5,97 | 81,0 |

Wie der Vergleich der Beispiele 1 und 2 zeigt, kann die Desaktivierung eines zur Synthese von Vinyloxiran aus Butadien und Sauerstoff eingesetzten Katalysators durch Eindosierung von Wasserdampf wieder rückgängig gemacht werden.

Beispiel 3 (nicht erfindungsgemäß)

Der Katalysator wurde mit einem Gasgemisch aus 20 Vol.-% 1,3-Butadien und 20 Vol.-% Sauerstoff, Rest Stickstoff, bei einer Temperatur von 220°C und bei Atmosphärendruck mit einer Raumgeschwindigkeit von 250 h$^{-1}$ belastet. Die Versuchsergebnisse sind in Tabelle 2 aufgeführt.

Tabelle 2

| Laufzeit [h] | Wasser [%] | U [%] | S [%] |
|---|---|---|---|
| 0,5 | 0 | 11,1 | 81,9 |
| 2,5 | 0 | 1,55 | 83,4 |
| 12,0 | 0 | 0,05 | 84,0 |

Beispiel 4 (erfindungsgemäß)

Der Katalysator wurde mit einem Gasgemisch aus 20 Vol.-% 1,3-Butadien, 20 Vol.-% Sauerstoff und 10 Vol.-% Wasserdampf, Rest Stickstoff, bei einer Temperatur von 220°C und bei Atmosphärendruck mit einer Raumgeschwindigkeit von 250 h$^{-1}$ belastet. Die Versuchsergebnisse sind in Tabelle 3 dargestellt.

Tabelle 3

| Laufzeit [h] | Wasser [%] | U [%] | S [%] |
|---|---|---|---|
| 0,5 | 10 | 6,7 | 82,4 |
| 2,5 | 10 | 8,9 | 81,3 |
| 12,0 | 10 | 5,9 | 84,5 |
| 24,0 | 10 | 9,1 | 81,1 |
| 146,0 | 10 | 6,0 | 84,1 |
| 243,0 | 10 | 6,0 | 83,8 |

Wie der Vergleich der Beispiele 3 und 4 in den Tabellen 2 und 3 zeigt, kann durch Eindosieren von Wasserdampf die Desaktivierung eines zur Herstellung von Vinyloxiran aus 1,3-Butadien und Sauerstoff verwendeten Katalysators verhindert werden, ohne daß die Selektivität für Vinyloxiran nennenswert beeinträchtigt wird.

**Patentansprüche**

1. Verfahren zur Herstellung von 3,4-Epoxy-1-buten durch die Gasphasenepoxidierung von 1,3-Butadien mittels Sauerstoff oder sauerstoffhaltigen Gasen an silberhaltigen Katalysatoren und Isolierung des 3,4-Epoxy-1-butens aus dem Reaktionsaustrag, dadurch gekennzeichnet, daß man die Gasphasenepoxidierung in Gegenwart von 6 bis 80 mol-% Wasserdampf, bezogen auf das dem Reaktor zugeführte Gasgemisch, durchführt.

2. Verfahren nach Anspruch 1, dadurch gekennzeichnet, daß man durch Verkokung desaktivierte silberhaltige Katalysatoren verwendet.

7

3. Verfahren nach Anspruch 1, dadurch gekennzeichnet, daß das 3,4-Epoxy-1-buten mit Wasser aus dem Reaktionsaustrag extrahiert wird.

4. Verfahren nach Anspruch 1, dadurch gekennzeichnet, daß das 3,4-Epoxy-1-buten mit Wasser einer Temperatur bis 100°C aus dem Reaktionsaustrag extrahiert wird.

5. Verfahren nach Anspruch 1, dadurch gekennzeichnet, daß nicht reagiertes 1,3-Butadien aus dem Reaktionsaustrag nach Abtrennung des 3,4-Epoxy-1-butens wieder in den Reaktor zur Herstellung von 3,4-Epoxy-1-buten zurückgeführt wird.

6. Verfahren nach Anspruch 1, dadurch gekennzeichnet, daß nicht reagiertes 1,3-Butadien aus dem Reaktionsaustrag nach Abtrennung des 3,4-Epoxy-1-butens und nach Abtrennung des im Reaktionsaustrag enthaltenen Kohlendioxids wieder in den Reaktor zur Herstellung von 3,4-Epoxy-1-buten zurückgeführt wird.

7. Verfahren nach Anspruch 1, dadurch gekennzeichnet, daß man die Gasphasenepoxidierung in Gegenwart von 2 bis 87 Vol.-%, bezogen auf das Volumen des dem Reaktor zugeführten Gasgemisches, eines $C_1$- bis $C_4$-Alkans ausführt.

8. Verfahren nach Anspruch 1, dadurch gekennzeichnet, daß man die Gasphasenepoxidierung in Gegenwart von 2 bis 87 Vol.-% Methan, bezogen auf das Volumen des dem Reaktor zugeführten Gasgemisches, ausführt.

9. Verfahren zur Reaktivierung von zur Herstellung von 3,4-Epoxy-1-buten durch die Gasphasenepoxidierung von 1,3-Butadien mittels Sauerstoff oder sauerstoffhaltigen Gasen verwendeter, durch Verkokung desaktivierter, silberhaltiger Katalysatoren, dadurch gekennzeichnet, daß man diese Katalysatoren bei Temperaturen von 150 bis 400°C mit Wasser und Sauerstoff oder Sauerstoff enthaltenden Gasen behandelt.

## Claims

1. A process for preparing 3,4-epoxy-1-butene by the gas phase epoxidation of 1,3-butadiene by means of oxygen or oxygen-containing gases over silver-containing catalysts and isolation of the 3,4-epoxy-1-butene from the reaction exit mixture, which comprises performing the gas phase epoxidation in the presence of from 6 to 80 mol% of water vapor, based on the gas mixture supplied to the reactor.

2. A process as claimed in claim 1 wherein silver-containing catalysts deactivated by coking are used.

3. A process as claimed in claim 1 wherein the 3,4-epoxy-1-butene is extracted from the reaction exit mixture with water.

4. A process as claimed in claim 1 wherein the 3,4-epoxy-1-butene is extracted from the reaction exit mixture with water at up to 100°C.

5. A process as claimed in claim 1 wherein unconverted 1,3-butadiene from the reaction exit mixture is after removal of the 3,4-epoxy-1-butene recirculated back into the reactor for the preparation of 3,4-epoxy-1-butene.

6. A process as claimed in claim 1 wherein unconverted 1,3-butadiene from the reaction exit mixture is after removal of the 3,4-epoxy-1-butene and after removal of the carbon dioxide present in the reaction exit mixture recirculated back into the reactor for the preparation of 3,4-epoxy-1-butene.

7. A process as claimed in claim 1 wherein the gas phase epoxidation is carried out in the presence of from 2 to 87% by volume, based on the volume of the gas mixture supplied to the reactor, of a $C_1$-$C_4$-alkane.

8. A process as claimed in claim 1 wherein the gas phase epoxidation is carried out in the presence of from 2 to 87% by volume of methane, based on the volume of the gas mixture supplied to the reactor.

9. A process for reactivating silver-containing catalysts which have been used for preparing 3,4-epoxy-1-butene by the gas phase epoxidation of 1,3-butadiene by means of oxygen or oxygen-containing gases and have become deactivated by coking, which comprises treating these catalysts at from 150 to 400°C with water and oxygen or oxygen-containing gases.

## Revendications

1. Procédé de préparation de 3,4-époxy-1-butène par l'époxydation en phase gazeuse de 1,3-butadiène au moyen d'oxygène ou de gaz contenant de l'oxygène en présence de catalyseurs contenant de l'argent et par l'isolement du 3,4-époxy-1-butène à partir du produit de la réaction, caractérisé en ce que l'on conduit l'époxydation en phase gazeuse en présence de 6 à 80% en moles de vapeur d'eau, par rapport au mélange gazeux introduit dans le réacteur.

2. Procédé selon la revendication 1, caractérisé en ce que l'on utilise des catalyseurs à l'argent désactivés par cokéfaction.

3. Procédé selon la revendication 1, caractérisé en ce que le 3,4-époxy-1-butène est extrait à partir du produit de la réaction avec de l'eau.

4. Procédé selon la revendication 1, caractérisé en ce que le 3,4-époxy-1-butène est extrait à partir du produit de la réaction avec de l'eau à une température allant jusqu'à 100°C.

5. Procédé selon la revendication 1, caractérisé en ce qu'après la séparation du 3,4-époxy-1-butène, le 1,3-butadiène n'ayant pas réagi contenu dans le produit de la réaction est renvoyé dans le réacteur pour la préparation de 3,4-époxy-1-butène.

6. Procédé selon la revendication 1, caractérisé en ce qu'après la séparation du 3,4-époxy-1-butène et après la séparation de l'anhydride carbonique contenu dans le produit de la réaction, le 1,3-butadiène n'ayant pas réagi contenu dans le produit de la réaction est renvoyé dans le réacteur pour la préparation de 3,4-époxy-1-butène.

7. Procédé selon la revendication 1, caractérisé en ce que l'on conduit l'époxydation en phase gazeuse en présence de 2 à 87% en volume d'un alcane en $C_1$-$C_4$ par rapport au volume du mélange gazeux introduit dans le réacteur.

8. Procédé selon la revendication 1, caractérisé en ce que l'on conduit l'époxydation en phase gazeuse en présence de 2 à 87% en volume de méthane par rapport au volume du mélange gazeux introduit dans le réacteur.

9. Procédé de réactivation de catalyseurs à l'argent, utilisés pour la préparation de 3,4-époxy-1-butène par l'époxydation en phase gazeuse de 1,3-butadiène au moyen d'oxygène ou de gaz contenant de l'oxygène et désactivés par cokéfaction, caractérisé en ce que l'on traite ces catalyseurs, à des températures de 150 à 400°C, par de l'eau et de l'oxygène ou des gaz contenant de l'oxygène.